# EUROPEAN PATENT SPECIFICATION

(11) **EP 2 797 679 B1**
(45) Date of publication and mention of the grant of the patent: **28.02.2018**
(21) Application number: 12862319.6
(22) Date of filing: 27.12.2012
(51) Int. Cl.: B01D 67/00, B01D 69/00, G01N 33/543

(54) **POROUS MEMBRANES HAVING A HYDROPHILIC COATING AND METHODS FOR THEIR PREPARATION AND USE**
PORÖSE MEMBRANEN MIT HYDROPHILER BESCHICHTUNG UND VERFAHREN ZU IHRER HERSTELLUNG UND VERWENDUNG
MEMBRANES POREUSES PRÉSENTANT UN REVÊTEMENT HYDROPHILE ET PROCÉDÉS POUR LEUR PRÉPARATION ET LEUR UTILISATION

(30) Priority: 30.12.2011 US 201113340793; 31.01.2012 US 201213362793
(43) Date of publication of application: 05.11.2014
(73) Proprietor: General Electric Company, Schenectady, NY 12345 (US)
(72) Inventor: LI, Bing, Niskayuna, New York 12309 (US); MOORE, David Roger, Niskayuna, New York 12309 (US)
(74) Representative: Aldenbäck, Ulla Christina
(86) International application number: PCT/US2012/071696
(87) International publication number: WO 2013/101857

(56) References cited:
- EP-A1- 2 797 680
- US-A1- 2007 272 607
- US-A1- 2008 200 434
- US-A1- 2009 188 857
- US-A1- 2009 208 975
- US-A1- 2010 143 611
- US-A1- 2011 147 308
- CHARLES ET AL.: 'Reduction of Non-Specific Protein Adsorption Using Poly(ethylene) Glycol (PEG) Modified Polyacrylate Hydrogels In Immunoassays for Staphylococcal Enterotoxin B Detection' SENSORS vol. 9, 2009, pages 645 - 655, XP055137760

## Description

### FIELD OF THE INVENTION

The present disclosure generally relates to methods for preparing porous membranes permanently grafted with a hydrophilic coating to minimize non-specific binding to porous membranes and use thereof for the immobilization of one or more specific biomolecules on the porous membrane for further analysis.

### BACKGROUND

Porous membranes, such as nitrocellulose membranes, are routinely used in a variety of processes, including biological applications that require the immobilization of one or more biomolecules. These biomolecules include but are not limited to proteins (e.g., antibodies) and nucleic acids (e.g., deoxyribonucleic acid (DNA) and ribonucleic acid (RNA)). Membranes able to both immobilize specific biomolecules of interest while at the same time minimizing non-specific binding of various molecules that interfere with the performance of, for example, *immunoassays, in vitro* diagnostic tests, particularly point-of-care diagnostic methods, and separation of analytes or biomolecules in biological samples (e.g., blood, urine, saliva, sputum, other bodily secretions, cells, and tissue samples) are desirable in the art. Such membranes would find use in a variety of biological processes and medical techniques. US2009/188857 discloses porous membranes (PVC) comprising a hydrophilic coating consisting of a hydrophilic additive and a hydrophilic polymer. PEGMA and TMPET have been suggested as hydrophilic additives. PEGMA and PEGDA have been used in immunoassays based on hydrogels prepared from PEG-modified polyacrylate (Charles, et. al., Sensors, vol. 9, 2009, pages 645-655). The immunoassays did not employ nitrocellulose membranes.

Nitrocellulose membranes exhibit an essentially non-specific interaction between the nitrocellulose membrane and biomolecule(s). Researchers have traditionally relied upon this passive association as the basis for the use of nitrocellulose membranes in a variety of "entrapment" type immobilization methods. Reliance on this passive interaction between a nitrocellulose membrane and a biomolecule of interest, however, leads to complications for successfully using nitrocellulose membranes in many biological applications. This technique necessarily limits the amount of the biomolecule that can be immobilized on the nitrocellulose membrane and, equally problematic, also permits non-specific binding of undesirable molecules (e.g., not the biomolecule of interest) to the nitrocellulose membrane. Reducing non-specific binding would allow for an increase in specific binding of the biomolecule of interest to the nitrocellulose membrane and also a decrease in the signal (e.g., desired binding of the desired biomolecule to the nitrocellulose membrane) to noise (e.g., non-specific binding of unwanted material to the nitrocellulose) ratio. Decreasing the signal to noise ratio would increase the performance and sensitivity of, for example, immunoassays, *in vitro* diagnostic tests, particularly point-of-care diagnostic methods, and separation methods of analytes or biomolecules from other materials in biological samples (e.g., blood, lymph, urine, saliva, sputum, other bodily secretions, cells, and tissue samples). A reduction in the signal to noise ratio in, for example, immunoassays is desirable in the art.

New compositions and methods of modifying (e.g., chemically modifying) porous membranes to improve immobilization and binding of biomolecules (e.g., proteins and nucleic acids) of interest to porous membrane substrates are needed in the art. Such modified porous membranes, including modified nitrocellulose membranes, would find use in, for example, immunoassays, *in vitro* diagnostic tests (e.g., point-of-care diagnostic applications), and techniques for the separation of biomolecules of interest in biological samples. Porous membranes, more particularly nitrocellulose membranes, coated with a compound to decrease non-specific binding to the membrane are needed in the art. Such membranes would improve the performance and sensitivity of, for example, numerous immunoassays.

New methods of modifying (e.g., chemically modifying) porous membranes (e.g., nitrocellulose membranes) to decrease non-specific binding of unwanted material to porous membrane substrates are needed in the art. Porous membranes, more particularly nitrocellulose membranes, coated with a compound to decrease non-specific binding to the membrane would be advantageous. Such membranes would improve the performance and sensitivity of, for example, numerous immunoassays by decreasing non-specific binding to the membranes, by potentially eliminating the need for traditional blocking agents used in, for example, immunoassays to minimize non-specific binding, and by increasing the signal to noise ratio relative to that observed in immunoassays performed with unmodified porous (e.g., nitrocellulose membranes).

### BRIEF DESCRIPTION

Production and use of modified porous membranes, particularly nitrocellulose membranes, are described herein comprising a polymeric hydrophilic coating bonded to the nitrocellulose membrane. The polymeric hydrophilic coating is generally permanently (e.g., covalently) bonded to the nitrocellulose membrane. The polymeric hydrophilic coating may be bonded to the nitrocellulose membrane by exposure to electron beam (e-beam) irradiation of the porous (e.g., nitrocellulose) membrane.

The production and use herein include a modified porous membrane such as a nitrocellulose membrane that comprises a polymeric hydrophilic coating typically permanently bonded to the membrane. The compositions find use in methods that rely on the binding of one or more biomolecule(s), such as proteins (e.g., antibodies) and nucleic acids (e.g., DNA or RNA), to porous membranes, including but not limited to, nitrocellulose membranes. The porous membranes, particularly nitrocellulose membranes, comprise a polymeric hydrophilic coating bonded to the membrane, wherein the membrane decreases non-specific binding of unwanted material to the porous membrane, more particularly a nitrocellulose membrane. The polymeric hydrophilic coating on the nitrocellulose membranes described herein include PEGMA, PEGDA or TMPET,

### DRAWINGS

The production and use of the chemically modified porous membranes will become better understood when the following detailed description is read with reference to the accompanying drawings in which like characters represent like parts throughout the drawings, wherein:
FIG. 1 is a schematic representation of the mechanism of a polymeric hydrophilic coating on a nitrocellulose membrane by e-beam irradiation.

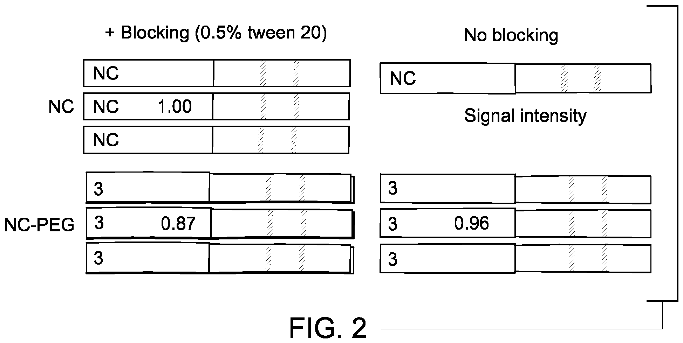

FIG. 2 provides the results of pregnancy tests using the modified porous membranes described herein. Details of the assays and an interpretation of the results is set forth in Example 4.

### DETAILED DESCRIPTION

The present invention is defined in its broadest sense by the appended claims.

Modified porous membranes, particularly nitrocellulose membranes, are used herein that comprise at least one polymeric hydrophilic coating bonded to the porous nitrocellulose membrane. The porous membranes, such as nitrocellulose membranes, are traditionally used to immobilize a biomolecule (e.g., DNA, RNA, or protein) on a porous solid substrate. The term "modified" as used herein, particularly in reference to the disclosed porous nitrocellulose membranes is intended to include any alteration to the membrane, for example, a chemical alteration, of the original, unmodified membrane. The "modified" porous nitrocellulose membranes used in the invention may be a nitrocellulose membrane comprising a polymeric hydrophilic coating grafted to the nitrocellulose membrane by electron-beam irradiation, as described below. The hydrophilic coating comprises a polyethylene glycol moiety, such as a PEGMA, a PEGDA, or a TMPET.

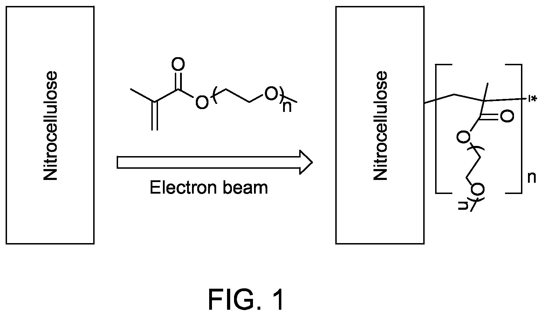

A schematic of exemplary modified porous membranes comprising a polymeric hydrophilic coating is provided below and set forth in FIG. 1. As shown in the diagram, in certain aspects, the porous membrane has the structure of Formula (I) that includes a polymeric hydrophilic coating grafted to a porous membrane (e.g., a nitrocellulose membrane), wherein the polymer hydrophilic coating comprises: 1) a polymer of a variable length of a chain monomers of an electron (e-beam) reactive moiety (designated as poly(A)ₓ, wherein x is the number of polymers present and ranges from one, two, three, four, and continuing to include all integers; 2) a linkage that forms a bond between (poly(A)ₓ); and 3) a functional group labeled B group which facilitates reaction with chemical groups, for example, an amine group present on a biomolecule of interest, thereby facilitating immobilization of a biomolecule on the porous membrane. The polymeric hydrophilic coating (e.g., labeled "poly(A)ₓ-linkage-B" in the schematic below) comprises several components (e.g., poly(A)ₓ polymer, a linkage, and a functional moiety B) and is grafted (e.g., covalently bond) to a porous membrane. See below and FIG. 1 for a more detailed description of the components and functions of the polymeric hydrophilic coating.

To more clearly and concisely describe and point out the subject matter of the claimed invention, the following definitions are provided for specific terms, which are used in the following description and in the appended claims.

The term "non-specific binding" as used herein refers to the attachment of a biomolecule on a porous a nitrocellulose membrane that is occurs by passive interaction of the biomolecule and the membrane and is independent of any particular, active interaction between the biomolecule and the membrane. "Non-specific binding" is also often referred to as "background binding" to a porous membrane, particularly a nitrocellulose membrane. One example of non-specific binding includes the attachment of a DNA molecule to a nitrocellulose membrane merely resulting from a random encounter in solution.

The term "modified" as used herein, particularly in reference to the disclosed porous nitrocellulose membranes, is intended to include any alteration to the membrane, for example, a chemical alteration, of the original, unmodified porous nitrocellulose membrane substrate. The "modified" porous membranes, more particularly nitrocellulose membranes, set forth herein may be modified (e.g., chemically modified) nitrocellulose membranes comprising a polymeric hydrophilic coating bonded to the nitrocellulose membrane. The polymeric hydrophilic coating comprises a PEG moiety, including a PEGMA, a PEGDA, or a TMPET.

Methods for preparing the porous nitrocellulose membranes having a polymeric hydrophilic coating bonded, typically permanently bonded, to the porous membranes are provided. In some embodiments, a polymeric hydrophilic coating is bonded onto a porous membrane such as a nitrocellulose membrane by providing an unmodified porous nitrocellulose membrane, immersing the membrane in in an aqueous solution of a hydrophilic compound, and exposing the membrane to e-beam radiation, thereby polymerizing the hydrophilic coating on the porous nitrocellulose membrane. For example, a nitrocellulose membrane is immersed in an aqueous solution of a hydrophilic compound, PEGMA, a PEGDA, or a TMPET, and then subjected to e-beam irradiation. Alternatively, in other aspects of the invention, the modified porous membranes are prepared by first subjecting a porous nitrocellulose membrane to e-beam irradiation followed by immersing the membrane in an aqueous solution of a hydrophilic compound, PEGMA, a PEGDA, or a TMPET. The methods of production of the modified porous nitrocellulose membrane substrates described herein that vary, for example, in the ordering of the method steps of immersing and the e-beam irradiation step are encompassed by the instant disclosure.

When used in the context of a method for preparing a modified porous membrane as described in greater detail below, the term "immersing" the porous membrane in an aqueous solution of, PEGMA, PEGDA, or TMPET, as recited in the claims, is generally accomplished by dipping the entire porous membrane, more specifically the nitrocellulose membrane, in the aqueous solution of the hydrophilic compound, PEGMA, PEGDA, or TMPET, and then removing any excess solution.

Nitrocellulose membranes are currently widely used in a variety of biological applications that require the immobilization of a particular biomolecule (e.g., DNA, RNA, or a protein such as an antibody) on a solid phase material. "Porous membrane" is intended to refer to, without limitation, to any porous membrane, including any commercially available or non-commercially available porous membrane, particularly a nitrocellulose membrane, more particularly a commercially available nitrocellulose membrane. In certain aspects of this disclosure, a nitrocellulose membrane is chemically modified to comprise, as set forth in FIG. 1, a polymeric hydrophilic coating of a PEG, wherein the polymeric hydrophilic coating decreases non-specific binding to the nitrocellulose membranes. Nitrocellulose membranes, which are made of a nitrocellulose polymer, have a strong affinity for DNA, RNA, and protein and prevent the denaturation of such biomolecules.

"Nitrocellulose membranes" as used in this application include all of those porous membrane products containing any nitrogen concentration, a diversity of pore sizes, and variable membrane thicknesses. In particular embodiments, the pore size of the porous membrane may be in the range of 0.01 to 50 microns. Moreover, pore diameter may be uniform throughout the porous membrane or, alternatively, pore diameter may be irregular. It is well within the skill and the knowledge of one in the art to select a nitrocellulose membrane, with the appropriate nitrogen content, pore size, and membrane thickness to achieve a specific, desired result. Moreover, the skilled artisan would immediately understand and appreciate the meaning of the phrase a "nitrocellulose membrane" and that such nitrocellulose membranes, include, for example, commercially available nitrocellulose membranes, may be "unbacked" membranes or alternatively contain a "backing material" or "backing support" such as a polyester (PE). The choice as to whether to use an "unbacked" or "backed" porous nitrocellulose membrane is dependent upon the particular application to be performed and is well within the purview of one of ordinary skill in the art to make such a selection.

Nitrocellulose membranes having any nitrogen concentration, pore size, or the presence or absence of a backing support are all encompassed in the term "nitrocellulose membrane" as used herein. Nitrocellulose membranes have a variety of chemical and physical properties and are routinely used in biological techniques that require, for example, the immobilization of a biomolecule of interest (e.g., DNA, RNA, or a protein such as an antibody) to a porous nitrocellulose membrane or for the collection of biomolecules on such membranes in order to separate them from other proteins, nucleic acids, and biomolecules or the like in a biological sample to be analyzed. Any nitrocellulose membrane may be utilized in the present disclosure.

One of skill in the art would be able to select a porous nitrocellulose membrane, appropriate for a particular method of use (e.g., an immunoassay). In certain aspects of the invention, a nitrocellulose membrane having a pore size in the range of 0.01 to 50 µm.

The term "biological sample" includes but is not limited to blood, serum, lymph, saliva, mucus, urine, other bodily secretions, cells, and tissue sections obtained from a human or non-human organism. Biological samples may be obtained by an individual undergoing the diagnostic test herself (e.g., blood glucose monitoring) or by a trained medical professional through a variety of techniques including, for example, aspirating blood using a needle or scraping or swabbing a particular area, such as a lesion on a patient's skin. Methods for collecting various biological samples are well known in the art.

"Immunoassay" is used herein in its broadest sense to include any technique based on the interaction between an antibody and its corresponding antigen. Such assays are based on the unique ability of an antibody to bind with high specificity to one or a very limited group of similar molecules (e.g., antigens). A molecule that binds to an antibody is called an antigen. Immunoassays can be carried out using either the antigen or antibody as the "capture" molecule to "entrap" the other member of the antibody-antigen pairing. As used herein, the term "immunoassay" further includes those assays that utilize antibodies for the detection of a non-protein biomolecule in a biological sample (e.g., metabolites of biochemical reactions).

An exemplary, albeit not exhaustive list of immunoassays includes a lateral flow assay (e.g., a home pregnancy test), a radioimmunoassay (RIA), an enzyme immunoassay (EIA), an enzyme-linked immunosorbent assay (ELISA), a fluorescent immunoassay, and a chemiluminescent immunoassay. The skilled artisan in the field possesses the skills needed to select and implement the appropriate method(s) for a particular situation, as well as the techniques for performing these immunoassays, as well as the skills to interpret the results. Immunoassays may produce qualitative or quantitative results depending on the particular method of detection selected.

The lateral flow assay is a common immunoassay, largely due to its ease of use, and includes such products as commercially available home-pregnancy tests and routine drug tests. Lateral flow assays are particularly advantageous because the devices and methods are generally simple to use and to interpret the test results, even by an individual with no formal medical training. Lateral flow devices and methods are intended to detect the presence or absence of a target analyte or biomolecule (e.g., human chorionic gonadotropin (hCG) in a lateral flow home pregnancy test) in a biological sample (e.g., urine). Although there is variation among lateral flow devices and assays, these tests are commonly used for home testing, point of care testing, and laboratory use. Lateral flow assays are often presented in a convenient "dipstick" format, as described in the examples below, in which the biological sample to be tested flows along a solid substrate (e.g., a porous membrane, often a nitrocellulose membrane) via capillary action. In certain formats of lateral flow assays, the dipstick is immersed in the biological sample, it encounters one or more reagents previously imprinted on the dipstick as the biological sample flows up the test strip, thereby encountering lines or zones on the test strip that have been previously imprinted with, for example, an antibody or antigen (e.g., hCG). When the biological sample encounters this reagent(s), a signal is generated to indicate whether the test is positive or negative for the presence of the analyte or biomolecule of interest (e.g., frequently a line visible to the naked eye as in the detection of hCG in a home pregnancy test indicative of the presence of hCG in the patient's urine).

Lateral flow devices and methods are well known in the art. See, for example, U.S. Patent Nos. 4,094,647; 4,313,734; 4,857,453; 5,073,484; 5,559,041; 5,571,726; 5,578,577; 5,591,645; 6,187,598; 6,352,862; and 6,485,982. Inclusion of a modified porous membrane of this disclosure, such as a modified nitrocellulose membrane comprising a polymeric coating of hydrophilic polymers, in known, for example, lateral flow devices and assays would significantly improve the performance, sensitivity, and specificity of such lateral flows devices and immunoassays, decrease the concentration of the analyte or biomolecule needed to obtain an accurate test results, and reduce the time to detect the presence or absence of the analyte or biomolecule, thereby minimizing the time required to acquire the test result.

All antibodies are proteins, more specifically glycoproteins, and exhibit binding specificity to an antigen (e.g., a portion of a polypeptide) of interest. The term "antibody" is used in the broadest sense and covers fully assembled antibodies, antibody fragments that can bind antigen (e.g., Fab', F'(ab)₂, Fv, single chain antibodies, diabodies), and recombinant peptides comprising the foregoing. "Antibody fragments" comprise a portion of an intact antibody, preferably the antigen-binding or variable region of the intact antibody. Examples of antibody fragments include Fab, Fab', F(ab')₂, and Fv fragments, diabodies, and linear antibodies (Zapata et al. (1995) Protein Eng. 8(10):1057 1062), single-chain antibody molecules, and multi-specific antibodies formed from antibody fragments. Any antibody or antibody fragment may be used in the practice of the invention.

In certain aspects of this invention, detection of antibody binding or immobilization on a solid phase material, including a nitrocellulose membrane, is needed. Any method known in the art for detecting antibody binding to a nitrocellulose membrane is encompassed by the disclosed invention. The determination and optimization of appropriate antibody binding detection techniques is standard and well within the routine capabilities of one of skill in the art. In some embodiments, detection of antibody binding can be facilitated by coupling the antibody to a detectable substance. Examples of detectable substances include various enzymes, prosthetic groups, fluorescent materials, luminescent materials, bioluminescent materials, and radioactive materials. Exemplary suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate (FITC), rhodamine, dichlorotriazinylamine fluorescein, dansyl chloride or phycoerythrin; a detectable luminescent material that may be couple to an antibody includes but is not limited to luminol; examples of bioluminescent materials include luciferase, luciferin, and aequorin; and examples of suitable radioactive material for detection of antibody binding include ¹²⁵I, ¹³¹I, ³⁵S, or ³H.

The modified porous membranes comprising a polymer coating of PEGMA. PEGDA or TMPET, may be further modified to comprise a hydrophilic compound immobilized on the porous membrane. The introduction of a hydrophilic compound onto the modified porous membrane comprising a polymeric coating may act as a blocking agent to decrease non-specific, background binding to the porous nitrocellulose membrane. Minimizing non-specific, background binding to a porous membrane improves the signal to noise ratio in, for example, immunoassays based on the specific interaction of an antibody immobilized on the porous membrane and a specific biomolecule of interest (e.g., a protein) in a sample being analyzed for the presence or quantity of this biomolecule.

In certain aspects of the invention, modified nitrocellulose membranes, are prepared as described above using an aqueous solution of a hydrophilic compound. The solution of the hydrophilic compound may further comprise a co-solvent to improve the solubility of the hydrophilic compound in water. For example, a surfactant, more particularly a non-ionic surfactant (e.g., polyoxyethylene (20) sorbitan monolaurate (Tween-20™)), may be used as a co-solvent to increase solubility of, for example, a PEG, in water. One of skill in the art will appreciate that the appropriate amount of a particular co-solvent (e.g., a nonionic surfactant such as polyoxyethylene (20) sorbitan monolaurate (Tween-20™) needed to increase the solubility of PEGMA, PEGDA, or TMPET must be determined and optimized experimentally.

The dosage of e-beam radiation used in the methods of grafting a polymer coating onto a porous nitrocellulose membrane, is selected to maximize the amount of the polymeric hydrophilic coating that is bonded to the nitrocellulose membrane while also limiting degradation of the porous nitrocellulose membrane known to result from e-beam irradiation. One of skill in the art will recognize that the appropriate dose of e-beam radiation used in the preparation of the modified porous membranes of the invention will need to be optimized experimentally. In particular embodiments, the dose of e-beam radiation used in the methods to prepare a modified porous membrane may be in the range of less than 1 kGy to approximately 50 kGy. The design of assays to optimize parameters such as the amount of the polymeric hydrophilic coating, optional surfactant, and the dose of e-beam radiation appropriate for use in the methods of the invention is standard and well within the routine capabilities of those of skill in the art.

The modified porous membranes find use in various biological applications that are dependent upon the immobilization of a biomolecule on a porous nitrocellulose membrane, including but not limited to immunoassays, *in vitro* diagnostic tests, and techniques for the isolation of a biomolecule of interest. Nitrocellulose membranes are of particular use in biological techniques because of their unique ability to immobilize nucleic acids (e.g., DNA and RNA) for use in Southern and Northern blots and for their binding affinity for amino acids (e.g., protein). As a result of these properties, nitrocellulose membranes are widely used as the substrate in diagnostic tests wherein antigen-antibody binding provides the test result (e.g., home pregnancy tests).

Although the ability of unmodified nitrocellulose membranes to bind biomolecules such as nucleic acids and proteins is beneficial, the modification of these membranes, decreasing non-specific binding to the nitrocellulose membrane facilitates the immobilization of biomolecules (e.g., DNA, RNA, and protein), provides significant advantages over unmodified porous membranes.

Accordingly, in certain aspects of the invention, a method is provided for determining the presence of an antigen in an immunoassay that uses a nitrocellulose membrane for immobilization of a biomolecule, wherein the nitrocellulose membrane used in the immunoassay is a nitrocellulose membrane comprising a polymeric hydrophilic coating of a PEGMA, a PEGDA or a TMPET bonded to the nitrocellulose membrane. Although the methods described herein can be used in the practice of any immunoassay, in certain embodiments the immunoassays include but are not limited to a lateral flow immunoassay, a radioimmunoassay, an enzyme immunoassay (EIA), an enzyme-linked immunosorbent assay (ELISA), a fluorescent immunoassay, and a chemiluminescent immunoassay.

Methods for improving the performance of an immunoassay are encompassed by this disclosure. The phrase "improving the performance of an immunoassay" is intended to include a variety of advantageous properties resulting from the use of the modified porous membranes, including but not limited to:
minimizing non-specific binding of unwanted materials to the modified porous membrane (alternatively referred to as a reduction in background binding), eliminating the need for the use of a blocking agent traditionally required for the performance of immunoassays, increasing the signal to noise ratio, and improving immobilization of a specific biomolecule of interest to the porous membrane by minimizing non-specific binding.

In one particular embodiment, a method for determining performance of an antigen in an immunoassay that uses a porous nitrocellulose membrane for immobilization of a biomolecule comprises providing a nitrocellulose membrane having a polymeric PEGMA PEGDA or TMPET hydrophilic coating covalently grafted to the porous membrane; immobilizing a first antibody that binds to an antigen of interest on the porous membrane; incubating a biological sample with a second antibody that specifically binds to the antigen or to the first antibody, wherein the second antibody is conjugated to a detectable substance; incubating the porous membrane comprising the first antibody immobilized on it with the biological sample comprising the second antibody; and thereby determining if the antigen is present in the biological sample by detecting if the second antibody binds to the porous membrane using the detectable substance bound to the second antibody. The skilled artisan would immediately appreciate that comprising washing the porous membrane following incubation of the membrane and the biological sample in order to remove unbound material. Moreover, an agent, such as a non-ionic surfactant (e.g., non-ionic surfactant Tween-20™ (e.g., polyoxyethylene (20) sorbitan monolaurate)) may be optionally used to wash the porous membrane.

As described in more detail above, a number of detectable substances may be conjugated to the second antibody. Exemplary detectable substances include but are not limited to enzymes, prosthetic groups, fluorescent dyes, luminescent materials, bioluminescent materials, a radioactive materials, and gold particles. Methods for conjugating or coupling a detectable substance to an antibody and for detecting these agents are well known in the art.

A method for decreasing non-specific binding in an immunoassay that uses a nitrocellulose membrane for immobilization of a biomolecule, wherein the nitrocellulose membrane used in the immunoassay is a nitrocellulose membrane comprising a polymeric hydrophilic coating bonded to the nitrocellulose membrane.

A method is disclosed for decreasing non-specific binding in an immunoassay that uses a nitrocellulose membrane for immobilization of a biomolecule, wherein the nitrocellulose membrane used in the immunoassay is a nitrocellulose membrane comprising a polymeric PEGMA, PEGDA or TMPET hydrophilic coating bonded to the nitrocellulose membrane. Moreover, a method is additionally provided for decreasing signal to noise ratio in an immunoassay that uses a nitrocellulose membrane for immobilization of a biomolecule, wherein the nitrocellulose membrane used in the immunoassay is a nitrocellulose membrane comprising a polymeric PEGMA, PEGDA or TMPET hydrophilic coating bonded to the nitrocellulose membrane.

The methods described above wherein a chemically modified porous nitrocellulose membrane comprises a polymeric hydrophilic PEGMA, PEGDA or TMPET membrane that decreases non-specific binding to the nitrocellulose membrane and also has the ability to bind a biomolecule such as DNA, RNA, or a protein imparts a number of advantages on immunoassays that utilize these modified porous membranes. For example, such membranes improve the performance and sensitivity of numerous immunoassays by decreasing non-specific binding to the membranes and by increasing the signal to noise ratio relative to that observed in immunoassays performed with unmodified porous membranes (e.g., nitrocellulose membranes), thereby permitting a more easily "observable" distinction between a positive and negative result in, for example an *in vitro* diagnostic assay.

A variety of immunoassays exist in the art, including those for drug testing, hormones, numerous disease-related proteins, tumor protein markers, and protein markers for cardiac injury. Immunoassays are also used to detect antigens on infectious agents such as *Hemophilus, Cryptococcus, Streptococcus,* Hepatitis B virus, HIV, Lyme disease, and Chlamydia trichomatis. These immunoassay tests are commonly used to identify patients with these and other diseases. Accordingly, compositions and methods for improving the sensitivity, specificity, and detection limits in immunoassays are of great importance in the field of diagnostic medicine.

The methods described herein may further permit the detection of one or more biomolecules in a biological sample (e.g., blood, serum, lymph, urine, saliva, mucus, bodily secretions, cells, or tissue). The biomolecules detected using the methods described here may be an antigen associated with a disease, such as a bacterial disease, a viral disease, or a fungal disease or a protein biomarker associated with diseases such as a cancer, a cardiac dysfunction, a heart attack, or an inflammatory disease.

The term "analyte" refers to a substance or chemical constituent whose presence or absence in, for example, a biological sample is being determined via an immunoassay or other diagnostic test.

"Biomolecule" as used herein refers without limitation to a nucleic acid (e.g., DNA or RNA) or a protein (e.g., an antibody) but further includes any organic molecule present in an organism (e.g., a human patient).

The following examples are offered by way of illustration and not by way of limitation:

### EXAMPLES

### Example 1: Bonding of a PEG on a Nitrocellulose Membrane

PEG derivatives, specifically PEGMA of molecular weights under of 300 and 2080 Da, polyethylene glycol diacrylate (PEGDA) of molecular weights under of 300 and 575 Da, and trimethylolpropane ethoxylate triacrylate (TMPET) of a molecular weight under 912 Da, were analyzed for their ability to bond to nitrocellulose when exposed to e-beam irradiation.

A dipping solution was prepared containing a PEG derivative monomer, with or without the non-ionic surfactant Tween-20™ (e.g., polyoxyethylene (20) sorbitan monolaurate), a non-ionic surfactant used to enhance the monomer solubility in water. The nitrocellulose membrane was immersed in an aqueous PEG solution and subjected to e-beam irradiation at a dose of 10 kGy or 50 kGy. The nitrocellulose membranes were washed with water thoroughly and then dried under vacuum overnight. Initial assessment on the grafting was measured by the weight gain of the nitrocellulose membrane following the above treatment. The results are set forth in Table 1.

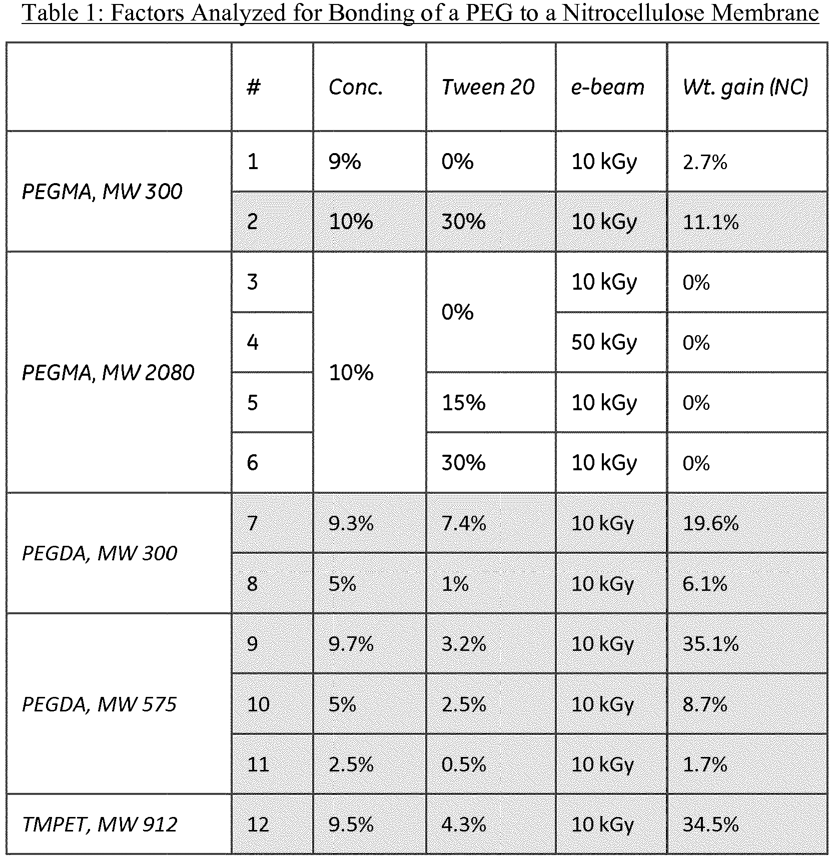

The grafting efficiency of PEGMA300 was enhanced by addition of polyoxyethylene (20) sorbitan monolaurate (Tween-20™) to the dipping solution. This increase in bonding efficiency of PEGMA300 to the nitrocellulose membrane was also confirmed by ATR-FTIR. Both PEGDA300 and PEGDA575 showed a linear relationship between grafting (e.g., bonding efficiency) and the monomer concentration used in the dipping solution. Polyoxyethylene (20) sorbitan monolaurate (Tween-20™) was used to improve solubility of the PEGDA300 and PEGDA575 at higher concentrations of the PEG monomer, with a weight gain of nitrocellulose-PEGDA membranes of up to 35.1% was achieved. TMPET 912 also showed good grafting of the PEG derivative on the nitrocellulose membrane with a 34.5% weight gain achieved. Grafting of the PEG derivatives was confirmed by IR analysis in accordance with standard methods in the art.

### Example 2: Nitrocellulose Grafted with a PEG Exhibits Decreased Non-Specific Binding

Assays were performed to determine if a nitrocellulose membrane bound with a polymeric PEG coating. A running buffer was prepared by coating 40 nm gold nanoparticles with a final concentration of 0.2 mg/ml BSA and an optical density of 0.8

Nitrocellulose membranes modified with PEG derivatives were prepared essentially as described above in Example 1. The modified nitrocellulose membranes were dipped into the running buffer, and the gold label was used to serve as the indicator of BSA presence of on the membranes.

The gold nanoparticles aggregated in the origin of the flow on the unmodified nitrocellulose membranes, suggestive of non-specific binding of BSA to the membrane, whereas the modified nitrocellulose membranes grafted with a PEG derivative the gold solution was able to flow smoothly up the membrane, and gold aggregates were only observed on the end (e.g., terminus) of the modified nitrocellulose-PEG membranes, strongly suggesting that the modified PEG-grafted membranes were able to block non-specific interaction between a protein (e.g., BSA) and the modified membrane. Blocking of non-specific protein binding was observed when as little as 1.7% PEGDA575 was grafted on the nitrocellulose membrane. Optimal blocking of non-specific binding was observed at PEG grafting efficiency around ∼10%.

### Example 3: PEG-Grafted Nitrocellulose Membranes Exhibit Reduced Non-specific Binding Similar to that Observed with Blocking Agents

Modified nitrocellulose membranes grafted with a PEG (e.g., PEGMA300 or PEGDA575) were assembled into a half stick lateral flow device, with the absorbent pad laminated on top of the modified nitrocellulose membrane, with an approximately 1 mm overlap, and the lateral flow device further supported by a polyester housing material with G&L 187 glue. Unmodified nitrocellulose membranes not grafted with any PEG served as a control.

A running buffer was prepared by coating 40 nm gold nanoparticles with a final concentration of 0.2 mg/ml BSA and an optical density of 0.8 OD. For purposes of comparison, a second running buffer was prepared by coating 40 nm gold nanoparticles with a final concentration of 0.2 mg/ml BSA and further comprising a 0.5% polyoxyethylene (20) sorbitan monolaurate (Tween-20™) solution, wherein the polyoxyethylene (20) sorbitan monolaurate (Tween-20™) served as a blocking agent. The half sticks were dipped into the above described running buffers, with or without polyoxyethylene (20) sorbitan monolaurate (Tween-20™), the capillary flow of the gold particles on the nitrocellulose membranes was monitored, and the backgrounds of the membrane were then analyzed by L-a-b colorimetric analysis, where "L, a, b" indicates "lightness, redness, greenness".

In those examples in which the running buffer contained blocking agent, all nitrocellulose membranes (modified or non-modified) were able to allow gold labeled BSA to flow smoothly from the bottom to the top, with all colors absorbed onto the absorbent pad.

When the running buffer containing the blocking agent was used, the BSA-labeled gold nanoparticles were trapped in the un-modified nitrocellulose membranes due to non-specific binding. The modified PEG-grafted nitrocellulose membranes, however, decreased non-specific binding to the membrane such that no visible color (e.g., gold) accumulated on the membranes after the BSA-labeled gold nanoparticles were allowed to completely flow through the membrane. When the color of background of the membranes was quantified, the modified PEG-nitrocellulose membranes exposed to the running containing no blocking agent (e.g., polyoxyethylene (20) sorbitan monolaurate (Tween-20™)) possessed a similar value to that observed with the unmodified nitrocellulose membrane exposed to the running buffer comprising the blocking agent polyoxyethylene (20) sorbitan monolaurate (Tween-20™). These results strongly suggest that the polymeric PEG coating on the modified nitrocellulose membranes functions in a similar and equivalent fashion to that of traditional blocking agents such as polyoxyethylene (20) sorbitan monolaurate (Tween-20™).

### Example 4: Nitrocellulose Membranes Grafted with a PEG Decrease Non-Specific Binding

Nitrocellulose membranes were printed with a test line (1 mg/ml anti-HCG-α) and a control line (0.5 mg/ml goat-anti-mouse-IgG), similar to a standard home pregnancy test, and then assembled into a half stick lateral flow device with absorbent pad laminated on top of nitrocellulose with an approximately 1 mm overlap, and the device further supported by a polyester housing material with G&L 187 glue.

For each half stick, 100 µl of running buffer containing 600 mIU/ml human chorionic gonadotropin (hCG; a hormone elevated in pregnancy), 1.5 ng/ml 40 nm gold nanoparticles coated with 0.15 mg/ml hCG-β was prepared. For comparison purposes, a second running buffer containing the blocking agent 0.5% polyoxyethylene (20) sorbitan monolaurate (Tween-20™) was also prepared. The half sticks were dipped in the running buffers for approximately 30 minutes to allow for completion of the assay. Signal intensities of the test line were quantified using Image J and normalized against the results obtained using unmodified nitrocellulose membranes in the presence of the blocking agent polyoxyethylene (20) sorbitan monolaurate (Tween-20™).

The signal intensity observed with the modified PEG-grafted nitrocellulose membranes was comparable to that seen with the unmodified membranes were exposed to running buffers comprising the blocking agent polyoxyethylene (20) sorbitan monolaurate (Tween-20™), strongly supporting that using modified nitrocellulose membranes comprising a polymeric hydrophilic (e.g., a PEG) coating may reduce or eliminate the need to include a traditional blocking agent like blocking agent polyoxyethylene (20) sorbitan monolaurate (Tween-20™) in lateral flow assays (e.g., home pregnancy tests) while maintaining the sensitivity of the diagnostic test.

## Claims

1. A method for determining the presence of an antigen in an immunoassay that uses a porous membrane for immobilization of a biomolecule comprising:
a) providing a porous nitrocellulose membrane having a PEGMA, a PEGDA, or a TMPET polymeric hydrophilic coating covalently grafted to the porous membrane;
b) immobilizing a first antibody that binds to an antigen of interest on the porous membrane;
c) incubating a biological sample with a second antibody that binds to the antigen, wherein the second antibody is conjugated to a detectable substance;
d) incubating the porous membrane comprising the first antibody immobilized on it with the biological sample comprising the second antibody; and
e) determining if the antigen is present in the biological sample by detecting if the second antibody binds to the porous membrane using the detectable substance bound to the second antibody.

2. The method of claim 1 further comprising washing the porous membrane following step (d) to remove unbound material.

3. The method of claim 2, wherein washing the porous membrane further comprises using a non-ionic surfactant.

4. The method of claim 3, wherein the detectable substance conjugated to the second antibody is selected from the group consisting of an enzyme, a prosthetic group, a fluorescent dye, a luminescent material, a bioluminescent material, a radioactive material, and gold

5. A method for preparing a nitrocellulose membrane comprising a polymeric hydrophilic coating bonded to the nitrocellulose membrane, comprising a PEGMA, a PEGDA, or a TMPET hydrophilic coating covalently grafted to the porous membrane comprising:
a) providing an unmodified nitrocellulose membrane;
b) immersing the nitrocellulose membrane in an aqueous solution of PEGMA, PEGDA or TMPET;
c) exposing the porous membrane to electron beam (e-beam) radiation; and
d) drying the nitrocellulose membrane, thereby preparing a nitrocellulose membrane comprising a polymeric hydrophilic coating bonded to the nitrocellulose membrane.

## Patentansprüche

1. Verfahren zum Bestimmen des Vorliegens eines Antigens bei einem Immunassay, bei dem eine poröse Membran zum Immobilisieren eines Biomoleküls verwendet wird, umfassend:
a) das Bereitstellen einer porösen Nitrocellulosemembran, die eine polymere, hydrophile PEGMA-, PEGDA- oder TMPET-Beschichtung aufweist, die kovalent auf die poröse Membran gepfropft ist;
b) das im Immobilisieren eines ersten Antikörpers, der sich an ein Antigen, das von Interesse ist, auf der porösen Membran bindet;
c) das Inkubieren einer biologischen Probe mit einem zweiten Antikörper, der sich an das Antigen bindet, wobei der zweite Antikörper an eine detektierbare Substanz konjugiert ist;
d) das Inkubieren der porösen Membran, die den ersten Antikörper darauf immobilisiert umfasst, mit der biologischen Probe, die den zweiten Antikörper umfasst; und
e) das Bestimmen, ob das Antigen in der biologischen Probe vorliegt, durch Detektieren, ob der zweite Antikörper sich an die poröse Membran bindet, unter Anwendung der detektierbar Substanz, die an den zweiten Antikörper gebunden ist.

2. Verfahren nach Anspruch 1, ferner das Waschen der porösen Membran auf den Schritt (d) hin, um ungebundenes Material zu entfernen.

3. Verfahren nach Anspruch 2, wobei das Waschen der porösen Membran ferner das Verwenden eines nichtionischen Tensids umfasst.

4. Verfahren nach Anspruch 3, wobei die detektierbare Substanz, die an den zweiten Antikörper konjugiert ist, aus der Gruppe ausgewählt wird bestehend aus einem Enzym, einer prosthetischen Gruppe, einem fluoreszierenden Farbstoff, einem biolumineszierenden Material, einem radioaktiven Material und Gold.

5. Verfahren zur Herstellung einer Nitrocellulosemembran umfassend eine polymere hydrophile Beschichtung, die an die Nitrocelluosemembran bondiert ist, umfassend eine hydrophile PEGMA-, PEGDA- oder TMPET-Beschichtung, die kovalent auf die poröse Membran gepfropft ist, umfassend:
a) das Bereitstellen einer unmodifizierten Nitrocellulosemembran;
b) das Eintauchen der Nitrocellulosemembran in eine wässrige Lösung von PEGMA, PEGDA oder TMPET;
c) das Aussetzen der porösen Membran Elektronenstrahl-(E-Strahl-) Strahlung gegenüber; und
d) das Trocknen der Nitrocellulosemembran, wodurch eine Nitrocellulosemembran hergestellt wird, die eine polymere hydrophile Beschichtung umfasst, die auf die Nitrocellulosemembran bondiert ist.

## Revendications

1. Procédé de détermination de la présence d'un antigène dans un immunodosage qui utilise une membrane poreuse pour l'immobilisation d'une biomolécule, comprenant les étapes consistant à :
a) fournir une membrane de nitrocellulose poreuse ayant un revêtement hydrophile polymère de PEGMA, de PEGDA ou de TMPET greffé par covalence sur la membrane poreuse ;
b) immobilier un premier anticorps qui se lie à un antigel d'intérêt sur la membrane poreuse ;
c) incuber un échantillon biologique avec un second anticorps qui se lie à l'antigène, dans lequel le second anticorps est conjugué à une substance détectable ;
d) incuber la membrane poreuse comprenant le premier anticorps immobilisé sur celle-ci avec l'échantillon biologique comprenant le second anticorps ; et
e) déterminer si l'antigène est présent dans l'échantillon biologique en détectant si le second anticorps se lie à la membrane poreuse en utilisant la substance détectable liée au second anticorps.

2. Procédé selon la revendication 1, comprenant en outre le lavage de la membrane poreuse à la suite de l'étape (d) pour éliminer la matière non liée.

3. Procédé selon la revendication 2, dans lequel le lavage de la membrane poreuse comprend en outre l'utilisation d'un tensioactif non ionique.

4. Procédé selon la revendication 3, dans lequel la substance détectable conjuguée au second anticorps est sélectionné dans le groupe constitué d'un enzyme, d'un groupement de prothèse, d'un colorant fluorescent, d'un matériau luminescent, d'un matériau bioluminescent, d'un matériau radioactif et de particules d'or.

5. Procédé de préparation d'une membrane de nitrocellulose comprenant un revêtement hydrophile polymère lié à la membrane de nitrocellulose, comprenant un revêtement hydrophile de PEGMA, de PEGDA ou de PMPET greffé par covalence sur la membrane poreuse, comprenant les étapes consistant à :
a) fournir une membrane de nitrocellulose non modifiée ;
b) immerger la membrane de nitrocellulose dans une solution aqueuse de PEGMA, de PEGDA ou de TMPET ;
c) exposer la membrane poreuse à un rayonnement de faisceau électronique (faisceau e) ;
d) sécher la membrane de nitrocellulose, en préparant de la sorte une membrane de nitrocellulose comprenant un revêtement hydrophile polymère lié à la membrane de nitrocellulose.
